# EUROPEAN PATENT SPECIFICATION

(11) **EP 2 456 842 B1**
(45) Date of publication and mention of the grant of the patent: **15.05.2019**
(21) Application number: 10855992.3
(22) Date of filing: 16.11.2010
(51) Int. Cl.: C09K 21/06

(54) **IONIC LIQUID FLAME RETARDANTS**
FLÜSSIGE IONISCHE FLAMMENHEMMER
RETARDATEURS D'INFLAMMATION DE TYPE LIQUIDE IONIQUE

(30) Priority: 09.08.2010 US 806267
(43) Date of publication of application: 30.05.2012
(73) Proprietor: Inovia Materials LLC, Greenwood Village, CO 80111 (US)
(72) Inventor: XU, Yanjie, Longmont, CO 80503 (US)
(74) Representative: Grund, Martin
(86) International application number: PCT/US2010/056874
(87) International publication number: WO 2012/021146

(56) References cited:
- CN-A- 101 608 348
- JP-A- 2007 039 820
- JP-B2- H0 774 336
- US-A1- 2004 099 178
- US-A1- 2008 194 831
- US-A1- 2009 043 016
- US-A1- 2010 108 956

## Description

### FIELD OF THE INVENTION

The present invention is broadly directed to novel flame or fire retardant compositions including ionic liquids.

### BACKGROUND OF THE INVENTION

Flame retardants are chemical additives which may be used across a variety of consumer products, such as plastics, textiles, leather, paper, rubber, etc. Chemicals which may be used as flame retardants can be mineral, halogen containing, nitrogen containing and phosphorus containing chemicals, silicon based chemicals, etc. The term "retardant" represents a class of use and not a class of chemical structure.

Preventive flame protection, including the use of flame retardants, has been practiced since ancient times. For example, alum was used to reduce the flammability by Egyptians at the time of about 540 BC. The advent of synthetic polymers earlier last century was of special significance, since the water soluble inorganic salts used up to that time were of little or no utility in these largely hydrophobic materials. Modern developments were, therefore, concentrated on the development of polymer compatible flame retardants. Wild forest fires comprise a serious problem, burning thousands of hectares all over the world each year. Diammonium phosphate (DAP), monoammonium phosphate (MAP), ammonium polyphosphate (APP) and ammonium sulphate (AS) have been used as long-term flame retardants. They are regarded as long-term flame retardants, because they can inhibit combustion even after the loss of their water matrix.

Fundamentally, four processes are involved in polymer flammability: preheating, decomposition, ignition and combustion and propagation. Flame retardants interfere with combustion during a particular stage of this process, i.e. during heating, decomposition, ignition or flame spread through physical or chemical actions.

There are several ways in which the combustion process can be retarded by physical action: for example cooling, formation of a protective layer/coating and/or dilution. During cooling action endothermic processes triggered by flame retardants may cool the material to a temperature below that required to sustain the combustion process. By formation of a protective layer/coating, a condensed combustible layer may be shielded from the gaseous phase with a solid or gaseous protective layer. A condensed phase is thus cooled, smaller quantities of pyrolysis gases are evolved, the oxygen necessary for the combustion process is excluded and heat transfer is impeded. By dilution, the incorporation of inert substances (e.g., fillers) and additives that evolve inert gases on decomposition may dilute the fuel in the solid and gaseous phases so that the lower ignition limit of the gas mixture is not exceeded.

Flame retardants may impede combustion by providing chemical reactions which interfere with combustion processes occurring in the solid and/or gas phases. For reactions in the gas phase, a free radical mechanism of a combustion process which takes place in the gas phase is interrupted by a flame retardant. Exothermic processes may thus be stopped, the system cools down, and the supply of flammable gases is reduced and eventually completely suppressed. For reactions in the solid phase, two types of reaction may take place. Firstly, breakdown of a polymer may be accelerated by a flame retardant, causing pronounced flow of a polymer and, hence, its withdrawal from the sphere of influence of the flame, which breaks away. Secondly, a flame retardant may cause a layer of carbon to form on a polymer surface. This can occur, for example, through the dehydrating action of the flame retardant generating double bonds in the polymer. These may form a carbonaceous layer by cyclizing and cross-linking.

In recent years, there are growing concerns about the safety of these flame retardant chemicals. An issue with the above mentioned forest flame retarding chemicals are their impact on the environment. Initially it was thought that these flame retardants would have no adverse on the environment, as their main active ingredients are agricultural fertilizers. However, ammonia, coming from the dissociation of the ammonium salts, is regarded as extremely toxic. Ecotoxicological studies were performed to understand the effects of long-term forest fire retardants on enzymatic activities, cells and microorganisms, thereby obtaining LC50 levels (lethal concentration). The LC50 value of ammonia is 0.53-4.94, which is extremely toxic. Toxicity studies on aquatic organisms relate the results obtained to the determined amount of flame retardants and ammonia. The data show that ammonia is the component that has most impact on these organisms under the testing conditions.

Brominated flame retardants, such as polybrominated diphenylethers (PBDEs), were first introduced into the consumer marketplace in the 1970s. They showed great compatibility with plastics and textiles, and offered superior flame retardant properties. Brominated flame retardants interrupt combustion by volatizing bromine radicals to react with high energetic free radicals O· and ·OH from the combustion, thereby preventing the spread of the flame. The most commonly used brominated flame retardants are PBDEs and tetrabromobisphenol A (TBBPA). By 2010, the brominated flame retardants market is projected to reach 1.7 billion pounds. Market Report by Peter Dufton; 2003.

Great efforts are being put into developing halogen free flame retardants, especially phosphorus based flame retardants. However, their flame retarding performance is not satisfactory. The prior art describes the use of some phosphonium ion salts. Doring et al describe polyphosphonium cations with selected anions as flame retardants in application US20100160476. Japanese patent application JP 2010163396 describes straight chain alkylaryl phosphonium salt structures as polymer dopants for high conductivity, heat resistance and flame retardancy. Tan et al, Faming Shuanli Shenqing Gongkai Shuomingshu, CAPLUS AN 2010:740737 (Patent), June 9, 2010, reported fireproofing agent containing quaternary phosphonium salt-modified montmorillonite as flame retardants. In US 2010/0108956 an electromagnetic interference (EMI) shielding material is described comprising a polymer film coated with a gel composition comprising a ionic liquid and fillers, e.g. SiO₂ or Al₂O₃. A flame retardant cellulose fiber treated with a flame retardant composition based on phosphorous and an ionic liquid is disclosed in CN 101 608 348. JP 2007 039820 discloses fibers treated with a fire retardant ionic liquid based on benzyldimethyl alkyl ammonium with a phosphate. US 2009/0043016 provides improved flame retardant polytrimethylene terephthalate compositions including a fluorinated sulfonate salt as a flame retardant additive. US 2008/194831 relates to ionic liquids of low viscosity and high electrochemical stability for the use *i.a.* as flame retardants. Long chain quaternary ammonium salts and their use as flame retardant agent for cellulosic material is described in JP H0774336. A review by Guo et al, Zhongguo Pige, 2004, CAPLUS AN 2005:551561 describes development and applications of tetrakis(hydroxymethyl) phosphonium salts as flame retardants for textiles, leather tanning agents, bactericides for wastewater treatment, etc ... Ammonium surfactants have been employed to modify the surface of nanoclays for flame retarding applications.

Despite health and environmental concerns, the world flame retardant chemicals market is projected to reach 5.7 billion pounds by the year 2012. The United States is the country with the tightest flame safety standards, and consequently the greatest use of brominated flame retardants. Nearly 98 percent of roughly 8,500 metric tons of PBDE used globally is consumed in US. However, brominated flame retardants are not chemically bound to the textiles and many substrates in plastic composites; therefore, they may easily escape into environment. There is growing concern over the persistence and bioaccumulation of brominated flame retardants and their risk to the environment and human health. Since brominated flame retardants are lipophilic and bioaccumulative substances, they may build up in fatty tissues once they enter a human or animal body. Studies have found bromated flame retardants to be widespread in the environment and in human tissues. Studies also have shown that these brominated flame retardants are toxic and can cause serious health disorders. In addition, women in North America have the highest levels globally of these chemicals in their breast milk.

The foregoing examples of the related art and limitations are intended to be illustrative and not exclusive. Other limitations of the related art will become apparent to those of skill in the art upon a reading of the specification and a study of the drawings or figures as provided herein.

### SUMMARY OF THE INVENTION

Therefore a continuing need exists for flame retardant compounds that are environmentally benign and nonmigrating. Ionic liquids show excellent resistance to migration and leaching and do not accumulate in fatty tissue causing toxicity. Additionally, incorporating biodegradable groups can make ionic liquids ready biodegradable and completely non-toxic.

The invention is defined by the appended claims.

In one embodiment, there is provided the use of a ionic liquid compound as a flame retardant in a material comprising 80 to 99.9 weight percent polymer, wherein said ionic liquid compound is blended with the polymer,the ionic liquid compound having the formula: 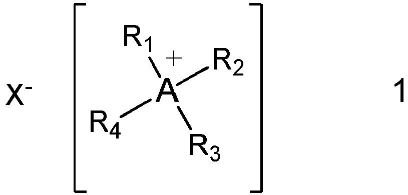 wherein A is selected from N or P; and wherein
i) when A is N, each R₁, R₂, R₃ and R₄ independently form a double bond with N and an adjacent R₁, R₂, R₃ or R₄ group or are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, cyano, -SMe, -SO₃H, -P((C₁-C₅)alkyl)₂ and -P(O)(OEt)₂; or wherein R₁, R₂ and R₃ together with N form a heteroaromatic or R₁ and R₂ together with N form a heterocyclic ring each unsubstituted or substituted by a group selected from halo, nitro, trifluoromethyl, trifluoromethoxy, methoxy, carboxy, -NH₂, -OH, - SH, -NHCH₃, -N(CH₃)₂, -SMe, cyano, (C₁-C₃)alkyl, aryl, (C₃-C₆)cycloalkyl, aryl(C₁-C₃)alkyl and heteroaryl; and
ii) when A is P, R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, cyano, -SMe, -SO₃H, -P((C₁-C₅)alkyl)₂ and -P(O)(OEt)₂; or
   wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, - OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃;
   provided that:
   a) when A is P, then R₁, R₂, R₃ and R₄ are not all hydroxymethyl;
   b) when A is N, X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, heteroaryl and heteroaryl(C₁-C₈)alkyl group that may be unsubstituted or substituted by one or two nitro, methoxy, carboxy, -NH₂, -OH, -SH, -NHCH₃, -N(CH₃)₂, -SMe and cyano;
   c) when A is N and at least one of R₁, R₂, R₃ and R₄ is -CH₃, then the material does not comprise of a clay; and
   d) when A is P, then X⁻ is selected from the group consisting of [PF₆]⁻, [NTf₂]⁻, [BR₅R₆R₇R₈]⁻, [BF₄]⁻, OH⁻, SCN⁻, SBF₆⁻, R₉PO₄⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, [Al₂Cl₇]⁻, [AlCl₄]⁻ oxalate, dicarboxylate and tricarboxylate, formate, phosphate, I⁻ and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano, and R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano; and provided that when the material is a resin and at least one of R₁, R₂, R₃ and R₄ is a -(CH₂)ₙ- group where n is 1 to 18, then X⁻ is not SBF₆⁻, PF₆⁻, BF₄⁻, AlF₆³⁻, CF₃SO₃⁻, AsF₆⁻, B[C₆F₅]₄⁻, B[C₆H₃(C₆H₃(CF₃)₂]₄⁻, B[C₆H₅]₄⁻, TiF₆²⁻, TiCl₅⁻, SnCl₅⁻, GeF₆²⁻, SiF₆²⁻, NiF₆²⁻ or ZrF₆²⁻;
and optionally, wherein X⁻ is a group as defined above that is bonded to the polymer.

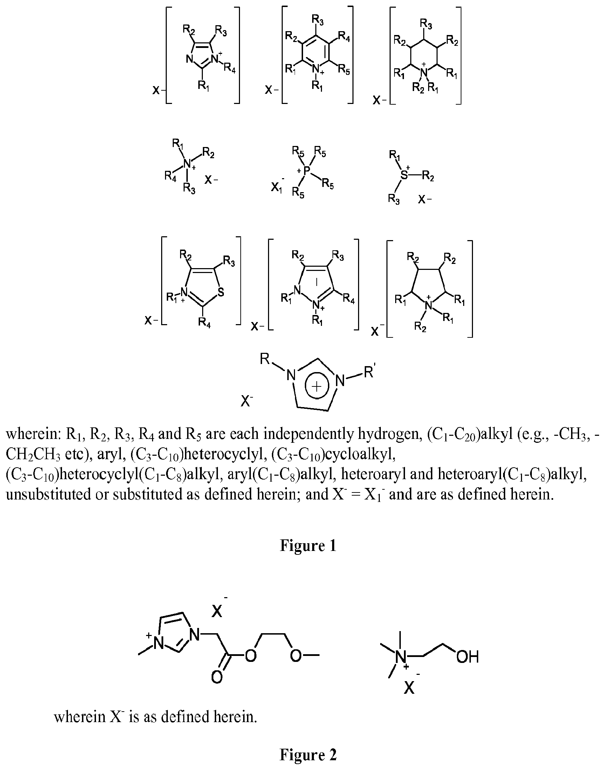

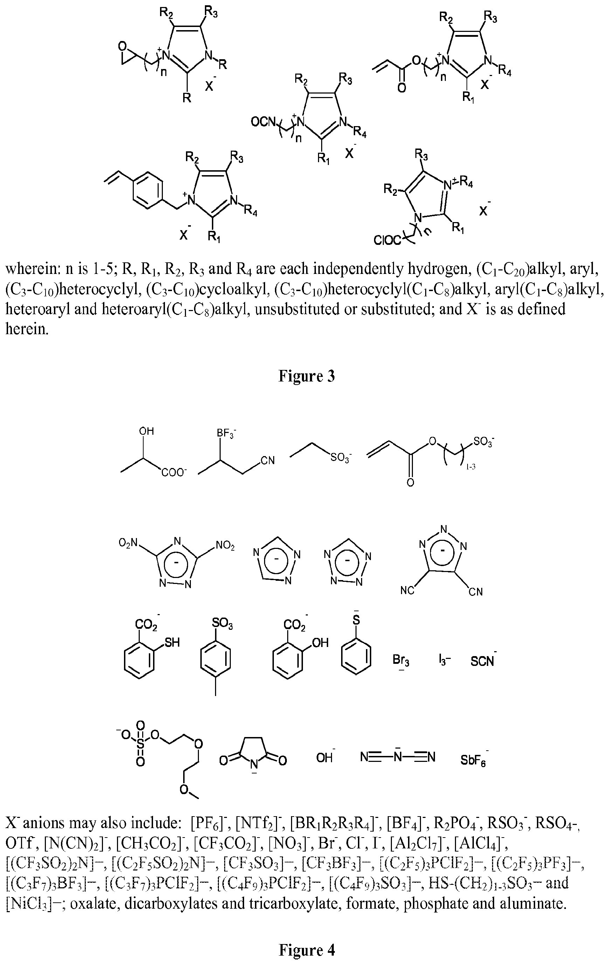

In one variation of the above ILs, X⁻ is selected from the group of anions as depicted in Figure 4. In another variation of the above, the ILs are selected from the group illustrated in Figures 1, 2 and 3, wherein X⁻ is as defined above. In a particular variation of the above, A is N, each R₁, R₂, R₃ and R₄ independently form a double bond with an adjacent R₁, R₂, R₃ or R₄ group. In one variation, when A is P, the material is a resin and at least one of R₁, R₂, R₃ and R₄ is a -(CH₂)ₙ- where n is 1 to 18, then X⁻ is not a Br⁻ and Cl⁻.

As disclosed herein, when A is N, each R₁, R₂, R₃ and R₄ independently form a double bond with an adjacent R₁, R₂, R₃ or R₄ group, means that when A is N, then N is bonded to R₁, R₂, R₃ and R₄, and together may form an acyclic ammonium compound, or a cyclic ammonium compound such as an imidazolium, pyridinium, pyridazinium or 1,2,4-triazolium. Similarly, when A is P, then P is bonded to R₁, R₂, R₃ and R₄ and together may form a a phosphonium compound. In one embodiment of the method, A⁺ together with R₁, R₂, R₃ and R₄ form a compound selected from the group consisting of an ammonium, imidazolium, guanidinium, pyridinium, pyridazinium, 1,2,4-triazolium, triazine, phosphazenium and phosphonium.

In another aspect of the above use, the compound is of the formula 2 or 9: 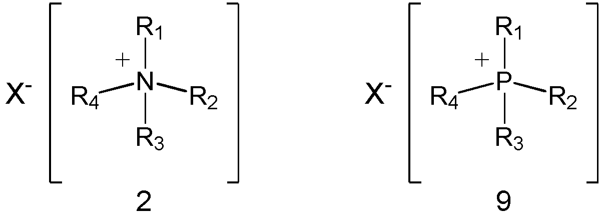
wherein for formula 2, each R₁, R₂, R₃ and R₄ independently form a double bond with N and an adjacent R₁, R₂, R₃ or R₄ group;
wherein for formula 2 and formula 9, R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe,-SO₃H, -P((C₁-C₅)alkyl)₂ and -P(O)(OEt)₂;
or for formula 2, wherein R₁, R₂ and R₃ together with N form a heteroaromatic or R₁ and R₂ together with N form a heterocyclic ring each unsubstituted or substituted by a group selected from halo, nitro, trifluoromethyl, trifluoromethoxy, methoxy, carboxy, -NH₂, -OH, - SH, -NHCH₃, -N(CH₃)₂, -SMe, cyano, (C₁-C₃)alkyl, aryl, (C₃-C₆)cycloalkyl, aryl(C₁-C₃)alkyl and heteroaryl; or
wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃; and
X⁻ is selected from the group consisting of [PF₆]⁻,[NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In another embodiment of the above use, the compound is of the formula: 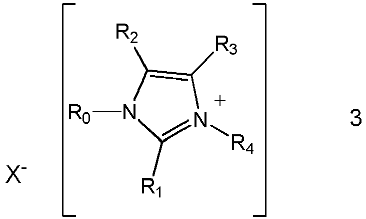 wherein:
Rₒ is selected from the group consisting of (C₁-C₅)alkyl and aryl that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, - P((C₁-C₅)alkyl)₂ and -P(O)(OEt)₂; and
R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, - SO₃H, -P((C₁-C₅)alkyl)₂ and -P(O)(OEt)₂; or
wherein at least one of R₀, R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In another embodiment of the above use, the compound is of the formula: 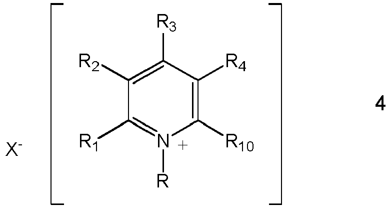
wherein R₁, R₂, R₃, R₄ and R₁₀ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe,-SO₃H, -P((C₁-C₅)alkyl)₂ and -P(O)(OEt)₂;
R is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, - N(CH₃)₂, cyano, -SMe, -SO₃H, -P((C₁-C₅)alkyl)₂ and -P(O)(OEt)₂; or
wherein at least one of R, R₁, R₂, R₃, R₄ and R₁₀ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl, - OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BP₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In another embodiment of the above use, the compound is of the formula 5 or 6: 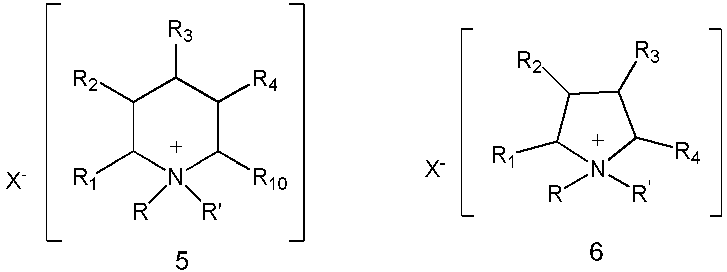
wherein R₁, R₂, R₃, R₄ and R₁₀ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe,-SO₃H, -P((C₁-C₅)alkyl)₂ and -P(O)(OEt)₂; or
R and R' are independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, cyano, -SMe, -SO₃H, -P((C₁-C₅)alkyl)₂ and -P(O)(OEt)₂; or
wherein at least one of R, R', R₁, R₂, R₃, R₄ and R₁₀ is selected from the group consisting of (C₁-C₁₀)alkyl substituted with one -Cl, -Br, -I, -CH=CH, -CH₂CH=CH, - epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BP₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In another embodiment of the above use, the compound is of the formula 7 or 8: 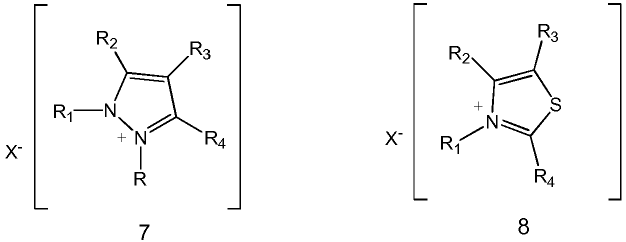 wherein:
R is selected from the group consisting of (C₁-C₅)alkyl and aryl that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, - P((C₁-C₅)alkyl)₂ and -P(O)(OEt)₂; and
R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, - SO₃H, -P((C₁-C₅)alkyl)₂ and -P(O)(OEt)₂; or
wherein at least one of R, R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In yet another aspect, each of R, R', R₀, R₁, R₂, R₃, R₄ and R₁₀ independently comprise of a reactive group that bonds the ionic liquid compound onto a polymer. The reactive group may include an alkene, acrylate, isocyanate, acid chloride, epoxide or other functional group that enables bonding to the polymer or other compounds.

According to another embodiment of the above use, the compound is of the formula: 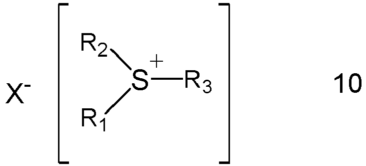
wherein R₁, R₂ and R₃ or are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, - SO₃H, -P((C₁-C₅)alkyl)₂ and -P(O)(OEt)₂; or
wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, - NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

In one embodiment for each of the above compound of formula 1, 2, 3, 4, 5, 6, 7, 8 or 9, at least one of R, R', R₀, R₁, R₂, R₃, R₄ and R₁₀ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃ may be polymerized into polymers to form polymers containing ILs.

In a particular variation of each of the above ILs of formulae 1, 2, 3, 4, 5, 6, 7, 8 and 9, X⁻ is selected from the group of anions as depicted in Figure 4.

In another embodiment of the above use, the use further comprises contacting or formulating the ionic liquid with a second different ionic liquid. In another embodiment of the use, the use further comprises contacting or formulating the material with an ionic liquid in combination with an agent selected from the group consisting of a mineral flame retardant, a halogenated flame retardant, a phosphorus based flame retardant, a nitrogen based flame retardant, a silicon based flame retardants and nanometric particles, and combinations thereof.

In another variation, the polymer is selected from the group consisting of a thermoplastic, phenolics, polycarbonates, polyurethanes, polyesters, polyethylene, polypropylene, polyacrylic acid, butadiene/acrylonitrile-acrylonitrile/styrene copolymers, ethylene-vinyl acetate copolymers, polyamides, acrylic resisn and epoxy resins. In another variation, the composition is a coating composition, a film, a composite material, an adhesive and a sealing composition.

Disclosed is a method of imparting a flame retarding property to a material comprising treating said material with an effective flame retarding amount of the composition of the formulas above in combination with a metal hydroxide, hydroxyl carbonate, borates the like.

Disclosed is also a method of imparting a flame retarding property to a material comprising treating said material with an effective flame retarding amount of the composition of the formulas above combined with a organic flame retardant. Disclosed is also a method of imparting a flame retarding property to a material comprising treating said material with an effective flame retarding amount of the composition of the formulas above combined with a halogenated flame retardant. Disclosed is also a method of imparting a flame retarding property to a material comprising treating said material with an effective flame retarding amount of the composition of the formulas above combined with halogenated flame retardant additives, halogenated monomers and copolymers which are reactive flame retardants, and the like.

Disclosed is also a method of imparting a flame retarding property to a material comprising treating said material with an effective flame retarding amount of the composition of the formulas above combined with a phosphorus based flame retardant. Disclosed is also a flame retardant composition comprising an ionic liquid combined with red phosphorus, inorganic phosphorus, organic phosphorus based compounds, intumescent flame retardant systems and the like.

Disclosed is also a method of imparting a flame retarding property to a material comprising treating said material with an effective flame retarding amount of the composition of the formulas above combined with a nitrogen based flame retardant Disclosed is also a method of imparting a flame retarding property to a material comprising treating said material with an effective flame retarding amount of the composition of the formulas above combined with silicon based flame retardants. Disclosed is also a method of imparting a flame retarding property to a material comprising treating said material with an effective flame retarding amount of the composition of the formulas above combined with silicones, silica and the like.

Disclosed is also a method of imparting a flame retarding property to a material comprising treating said material with an effective flame retarding amount of the composition of the formulas above combined with nanometric particles. Disclosed is also a method of imparting a flame retarding property to a material comprising treating said material with an effective flame retarding amount of the composition of the formulas above combined with a nanoclay, carbon nanotubes, nanoscale particulate additives.

Disclosed is also a method of imparting a flame retarding property to a material comprising treating said material with an effective flame retarding amount of the composition of the formulas above also functioning as a dispersant. Disclosed is also a method of imparting a flame retarding property to a material comprising treating said material with an effective flame retarding amount of the composition of the formulas above also functioning as a plasticizer.

Disclosed is also a method of imparting a flame retarding property to a material comprising treating said material with an effective flame retarding amount of the composition of the formulas above also functioning as an antibacterial. Disclosed is also a method of imparting a flame retarding property to a material comprising treating said material with an effective flame retarding amount of the composition of the formulas above also functioning as a lubricant. Disclosed is also a method of imparting a flame retarding property to a material comprising treating said material with an effective flame retarding amount of the composition of the formulae above that also function as an anti-corrosion agent.

### DETAILED DESCRIPTION OF THE INVENTION

### DEFINITIONS:

Unless specifically noted otherwise herein, the definitions of the terms used are standard definitions used in the chemical arts. Exemplary embodiments, aspects and variations are illustratived in the figures and drawings, and it is intended that the embodiments, aspects and variations, and the figures and drawings disclosed herein are to be considered illustrative and not limiting.

In one embodiment, the group that is an alkyl, aryl, heterocyclyl, (C₁-C₈)cycloalkyl, hetrocyclyl(C₁-C₈)alkyl, aryl(C₁-C₈)alkyl, heteroaryl or heteroaryl(C₁-C₈)alkyl group may be substituted or unsubstituted.

An "alkyl" group is a straight, branched, saturated or unsaturated, aliphatic group having a chain of carbon atoms, optionally with oxygen, nitrogen or sulfur atoms inserted between the carbon atoms in the chain or as indicated. A (C₁-C₂₀)alkyl, for example, includes alkyl groups that have a chain of between 1 and 20 carbon atoms, and include, for example, the groups methyl, ethyl, propyl, isopropyl, vinyl, allyl, 1-propenyl, isopropenyl, ethynyl, 1-propynyl, 2-propynyl, 1,3-butadienyl, penta-1,3-dienyl, penta-1,4-dienyl, hexa-1,3-dienyl, hexa-1,3,5-trienyl, and the like. An alkyl group may also be represented, for example, as a -(CR¹R²)ₘ- group where R¹ and R² are independently hydrogen or are independently absent, and for example, m is 1 to 8, and such representation is also intended to cover both saturated and unsaturated alkyl groups.

An alkyl as noted with another group such as an aryl group, represented as "arylalkyl" for example, is intended to be a straight, branched, saturated or unsaturated aliphatic divalent group with the number of atoms indicated in the alkyl group (as in (C₁-C₂₀)alkyl, for example) and/or aryl group (as in (C₅-C₁₄)aryl, for example) or when no atoms are indicated means a bond between the aryl and the alkyl group. Nonexclusive examples of such group include benzyl, phenethyl and the like.

An "alkylene" group is a straight, branched, saturated or unsaturated aliphatic divalent group with the number of atoms indicated in the alkyl group; for example, a -(C₁-C₃)alkylene- or -(C₁-C₃)alkylenyl-.

A "cyclyl" such as a monocyclyl or polycyclyl group includes monocyclic, or linearly fused, angularly fused or bridged polycycloalkyl, or combinations thereof. Such cyclyl group is intended to include the heterocyclyl analogs. A cyclyl group may be saturated, partially saturated or aromatic.

The term "cellulose" or "cellulose fiber" generally refers to a fiber composed of, or derived from, cellulose, a main component of the cell walls of plants. Examples of cellulose or cellulosic fibers include cotton, rayon, linen, jute, hemp and cellulose acetate.

The term "flame resistant" is used to describe a material that burns slowly or that is self-extinguishing after removal of an external source of ignition. For example, as the term relates to fabric or textiles, a fabric or yarn may be flame resistant because of the innate properties of the fiber, the fabric construction, or the presence of flame retardant compounds or formulations applied to the fabric.

The term "flame retardant" or "flame retardant compound" as it relates to textiles or fabric, refers to a compound that may be applied as a topical treatment to a fiber, fabric, or other textile item during processing to reduce its flammability. In some aspects, flame retardant chemicals are applied to the already constructed fabric substrate to produce a flame resistant fabric.

"Halogen" or "halo" means fluorine, chlorine, bromine or iodine.

A "heterocyclyl" or "heterocycle" is a mono-cycloalkyl or bi-cycloalkyl wherein one or more of the atoms forming the ring or rings is a heteroatom that is a N, O, or S. Nonexclusive examples of heterocyclyl include piperidyl, 4-morpholyl, 4-piperazinyl, pyrrolidinyl, 1,4-diazaperhydroepinyl, 1,3-dioxanyl, and the like. In one aspect, the heterocyclyl may also include carbohydrate-based compounds, such as glucose. Accordingly, the ILs of the present application includes sugar-derived ILs, including glucose-derived ILs. Such glucose derived ILs include 1,5-anhydro-2,3,4-tri-O-methyl-D-glucitol-6-O-triethylammonium trifluoromethanesulfonate, 1,5-anhydro-2,3,4-tri-0-methyl-D-glucitol-6-O-diethylsulfonium trifluoromethanesulfonate and 1,5-anhydro-2,3,4-tri-O-methyl-D-glucitol-6-O-tetrahydrothiophenyl trifluoromethanesulfonate.

Salts include acid addition salts formed with inorganic acids such as hydrochloric acid, hydrobromic acid, sulfuric acid, phosphoric acid, and the like; or with organic acids such as acetic acid, propionic acid, hexanoic acid, malonic acid, succinic acid, malic acid, citric acid, gluconic acid, salicylic acid and the like.

"Substituted or unsubstituted" or "optionally substituted" means that a group such as, for example, alkyl, aryl, heterocyclyl, (C₁-C₈)cycloalkyl, hetrocyclyl(C₁-C₈)alkyl, aryl(C₁-C₈)alkyl, heteroaryl, heteroaryl(C₁-C₈)alkyl, and the like, unless specifically noted otherwise, may be unsubstituted or, may substituted by 1, 2 or 3 Substituents selected from the group such as halo, nitro, trifluoromethyl, trifluoromethoxy, methoxy, carboxy, -NH₂, -OH, -SH, -NHCH₃, -N(CH₃)₂, -SMe, cyano and the like.

The ILs of the present application may be racemic compounds or may be chiral substantially enantiomeric or diastereomeric pure or mixtures thereof.

The present disclosure may be understood by reference to the following detailed description taken in conjunction with the drawings described below.

To replace brominated flame retardants and other chemical compounds that may have toxic bioaccumulative effects; a different class of materials, namely ionic liquids ("IL"), are used for the purpose of flame retarding.

An ionic liquid is a salt in which the ions are poorly coordinated as is well known in the art. At least one ion in the salt has a delocalized charge and one component is organic, which prevents the formation of a stable crystal lattice.

Ionic liquids have capabilities to form a wide range of intermolecular interactions that include strong and weak ionic, hydrogen boding, van der Waals, dispersive, pie-pie interactions. Ionic liquids exhibit compatibility with a wide variety of materials including salts, fats, proteins, amino acids, surfactants, oils, inks and plastics, and even DNA. Ionic liquids have been intensively studied for many applications, such as solvents, catalysts, separation, extraction, biomass processing, etc. ILs have been used as plasticizers, dispersants, and lubricants. When used as plasticizers, they show excellent resistance to migration and leaching which mitigates one of the most significant issues with current flame retardant compounds.

Ionic liquid flame retardants may be suitably configured by selection of cations and anions chosen from, but not limited to, those shown in Figures 1, 3 and 4.

Ionic liquids are compounds which may contain halogen, nitrogen, phosphorus, sulfur, or some combination of these elements. Ionic liquid compounds may be designed with halogen, nitrogen, sulfur, phosphorus or some combinations of these elements, and so the compounds may be used as flame retardants, either though physical action or chemical action to inhibit combustion processes as discussed above.

Due to the large number of possible combinations of ion pairs, the ability to select the physical and chemical properties of possible ionic liquid flame retardants is essentially unlimited. Functionalization of a ligand or "head", such as by changing the length of a ligand R group, adding a ligand to different positions of a head, and/or adding a halogen to a ligand or head further increases the number of possible ionic liquid flame retardants. The head may be defined as the positively charged core atom or ring of the cation species of the ionic liquid.

In one aspect, ionic liquids are modified to design biodegradable and nontoxic ionic liquids via incorporation of ethereal side chains. One such example is shown in Figure 2. Greener Solvents; Room Temperature Ionic Liquids from Biorenewable Sources, Scott Handy, Chem. Eur. J. 2003, 9, 2938-2944.

In another embodiment incorporation of reactive groups into ligands, produces ionic liquids which may be chemically bound with a substrate to impart flame retarding properties to substrates. Five such examples are shown in Figure 3. Other reactive groups may include, but are not limited to hydroxyl and/or carboxyl groups.

In another embodiment, ionic liquids may be formulated with other ionic liquids, or traditional flame retardants or additives. These traditional flame retardants can be mineral flame retardants, halogen containing flame retardants, phosphorous based flame retardants, nitrogen based flame retardants, silicon based flame retardants, nanometric particles, etc. Mineral flame retardants can be metal hydroxides, hydroxycarbonates, borates, etc.; halogen containing flame retardants can be halogen flame retardant additives, reactive halogenated flame retardant monomers or polymers; phosphorous based flame retardants can be red phosphorous, inorganic phosphate, organic phosphorous based compounds, etc.; silicon based flame retardants can be silicon, silica compounds, etc.; nanometric particles can be nanoclay, carbon nanotube, nanoscale particulate additives, etc.

Ionic liquids may also be used as multifunctional additives. For example, an ionic liquid may be used as a lubricant and flame retardant, a plasticizer and flame retardant, a dispersant and flame retardant, and an antibacterial agent and flame retardant.

The proposed flame retardants can be used in many fields including plastics, textiles, paper, leather, wood, etc. In addition, the flame retardants of the present application can also be used as flame retardants for fighting forest fires.

### DESCRIPTION OF THE FIGURES

Figure 1 provides a list of representative ILs depicted in Formula I, including cyclic and acyclic ILs. In one variation, R and R' are each independently selected from the group consisting of (C₁-C₂₀)alkyl (inclung -CH₃, -CH₂CH₃, -allyl, -propargyl, etc ...), aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -OR, - SH, -NHCH₃, -N(CH₃)₂, cyano, -SMe, -Si(OR)₃, -SO₃⁻, -SO₃H, -CO₂R, -P((C₁-C₅)alkyl)₂ and -P(O)(OEt)₂, wherein R is (C₁-C₂₀)alkyl, (C₃-C₁₀)cycloalkyl and aryl.
Figure 2 illustrates examples of cyclic and acyclic ILs, including an imidazolium IL and a choline based IL.
Figure 3 provides representative examples of heterocyclic ILs, including imidazolium ILs monomers that have functional groups.
Figure 4 provides a list of representative examples of the anion X⁻ of the ILs.
Figure 5 is a depiction of an apparatus for conducting a flammability test.

### EXAMPLES

The materials and reagents used are either available from commercial suppliers or are prepared by methods well known to a person of ordinary skill in the art, following procedures described in such references as Fieser and Fieser's Reagents for Organic Synthesis, vols. 1-17, John Wiley and Sons, New York, N.Y., 1991; Rodd's Chemistry of Carbon Compounds, vols. 1-5 and supps., Elsevier Science Publishers, 1989; Organic Reactions, vols. 1-40, John Wiley and Sons, New York, N.Y., 1991; March J.: Advanced Organic Chemistry, 4th ed., John Wiley and Sons, New York, N.Y.; and Larock: Comprehensive Organic Transformations, VCH Publishers, New York, 1989.

In one embodiment, ionic liquids of the present application are further modified by the incorporation with ethereal side chains to provide biodegradable and nontoxic ionic liquids. One such example is shown in Figure 2. Greener Solvents; Room Temperature Ionic Liquids from Biorenewable Sources, Scott Handy, Chem. Eur. J. 2003, 9, 2938-2944.

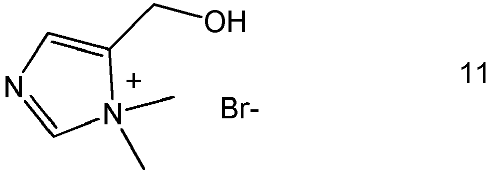

Hydroxymethyl imidazolium ionic liquid derivatives may be synthesized from fructose according to the method reported by Totter and Handy in Room Temperature Ionic Liquids: Different Classes and Physical Properties; Scott Handy; Current Organic Chemistry, 2005, 9, 959-988; Organic Letter, 2003, Vol. 5, No. 14, pp 2513-2515, Handy et al; Organic Syntheses, Coll. Vol. 3, p.460 (1955); Vol. 24, p.64 (1944), Totter et al.

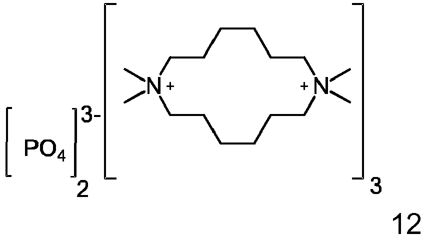

The cyclic diaminophosphate compound above may be prepared according to chemistry described by Lall et al in Chem. Comm., 2000, 2413-2414.

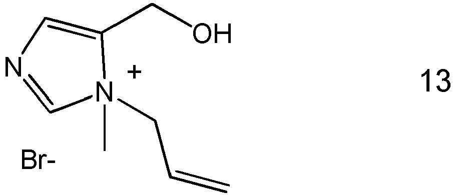

The allyl imidazolium bromide may be prepared according to chemistry described by Liu et al in Science of China, Series B: Chemistry, 2006, 149, 1, 385-401.

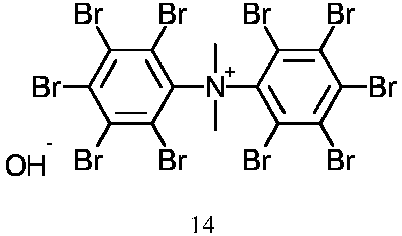

The brominated biphenylammonium compound above may be prepared by methylation of the brominated biphenylamine described in Czech patent 233407 titled, "Preparation of brominated diphenyl amines as fire proofing agents."

### Treatment of Polymers and Resins with IL Flame Retardants:

The polymer comprises of about 80 to 99.9 weight percent of the composition that is blended with the IL, and optionally, additive, as provided herein. In one variation, the polymer is a polyolefin. In one variation of the polymer composition, the polyolefin is selected from polypropylene and polyethylene, such as isotactic, atactic and syndiotactic polypropylene, HDPE, LDPE and LLDPE, random and heterophasic copolymers of propylene, ethylene, butene, hexene and octane. In another variation, the polymer is selected from at least one of polyesters, epoxy resins, ABS combinations, halogenated polymers, polyethylene, polystyrene, silicones, silicone rubbers, ethyl vinyl acetate, and their copolymers.

In one aspect of the present application, the polymer is a resin. Such resin may include thermoplastic resin, thermoset resin, thermoplastic resin blend or thermoset resin blend. In one variation, the resin may be selected from polycarbonates, polyamides, polyesters, blends of polycarbonates with other polymers, polyphenylene ether, polyphenyleneoxide, blends of polyphenylene ether with styrenics, blends of polyphenyleneoxide with styrenic materials, polyaramids, polyimides, styrenic materials, polyacrylates, styrene-acrylonitrile resins, halogenated plastics, polyketones, polymethylmethacrylate (PMMA), thermoplastic elastomers, cellulosics, rayon or polylactic acid. In another variation, the polymer employed may be polycarbonates, polycaprolactam, polylauryllactam, polyhexamethyleneadipamide, polyhexamethylenedodecanamide, blends of Nylons with other polymers, polybutylene terephthalate, polyethylene terephthalate, polyethylene naphthalate, polycarbonate-acrylonitrile-butadiene-styrene blends, polycarbonate-polybutylene terephthalate blends, polyphenylene ether, polyphenyleneoxide, polyphenylene sulfide, polyether sulphone, polyethylene sulfide, acrylonitrile-butadiene-styrene, polystyrene, styrene-acrylonitrile resins, polyvinyl chloride, fluoroplastics, polymethylmethacrylate, thermoplastic urethanes, thermoplastic vulcanizates, or styrene ethylene butylene styrene copolymer.

The resins of may be uncured resins that have no curing agent, semi-cured resins or cured resins. In a particular variation, the amount of the IL that may be incorporated into the resins of the present application may be about 0.01 to about 30% by weight, about 0.01 to about 20% by weight %, about 0.01 to about 10% by weight or about 0.01 to about 5% by weight.

### EVA Copolymers:

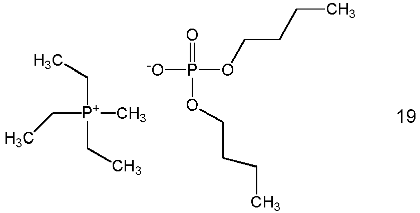

Treatment of ethylene-vinyl acetate (EVA) copolymer with Triethylmethylphosphonium dibutyl phosphate:
EVA (80 g), IL **19** (3 g), low melting glass (5 g) and ATH (alumina trihydrate, 12 g) are mixed, melt blended in a Thermo Haake Rheomix with a screw speed of 60 rpm, and the mixing time is 15 min for each sample. The mixed samples are transferred to a mold and preheated at 180 °C for 5 min and then pressed at 15 MPa, followed by cooling the samples to room temperature while maintaining the pressure for 5 min.

### Thermosets with ILs:

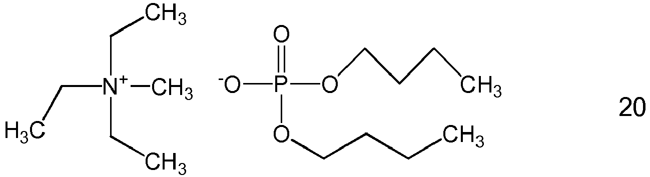

Phenolic resin (80 g), IL **20** (3 g), glass flake (5 g) and ATH (12 g) are mixed and compounded in a similar manner as described above. The polymers prepared according to the above procedure are found to have flame retardant properties.

### Thermoplastics containing ILs:

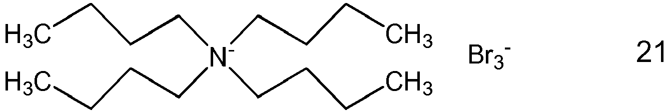

Polybutylene terephthalate (90 g), IL **21** (7 g), antimony trioxide (3 g) are mixed and extruded in a similar manner as described above. The polymers obtained according to the above procedure are found to have flame retardant properties.

### Polycarbonate Polymers with IL:

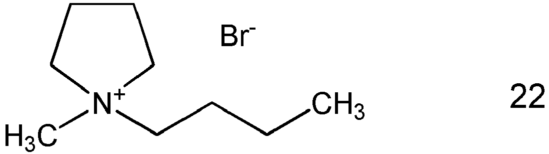

Polycarbonate (90 g), IL **22** (5 g), silicon (3 g) and SnCl₂ (2 g) are mixed and extruded in a similar manner as described above. The polymers obtained according to the above procedure are found to have flame retardant properties.

### Incorporation of Functionalized ILs into Polymers:

ILs monomers that have functional groups such as -Cl, -Br, -I, -CH=CH, - CH₂CH=CH, -epoxide, -OC(O)-CH=CH, -NCO, -C(O)Cl, -C(O)Br, -C(O)-imidazolyl, - CO₂(C₁-C₃)alkyl, -OC(O)CH₂C(O)CH₃ and -CH=CR₁₀CO₂(C₁-C₃)alkyl where R₁₀ is H or CH₃ may be polymerized into polymers to form polymers containing ILs. For example, ILs containing ethylene oxide groups may be polymerized by initiation with different agents such as postassium t-butoxide in a solvent, such as DMF.

Functionalized ILs Modified Rubber: The modified rubber may be a rubber phase polymer in a matrix containing functionalized ILs. The modified rubber may be prepared by polymerizing the functionalized IL with various rubbers. The modified rubber may be prepared by standard methods such as emulsion polymerization, suspension polymerization, bulk polymerization and by extrusion of a graft copolymer resin and a functionalized IL. The polymerization method employed may provide the modified rubber in about 50% to 90% by weight. In one embodiment, the functionalized ILs may be employed in about 1% to about 30% by weight with the rubber polymer in about 50% to about 95% by weight. Optionally, a copolymerizable polymer may be added in an amount of about 10% to about 30% by weight. Suitable polymers that may be employed include polybutadiene, poly(styrene-butadiene), poly(acrylonitrile-butadiene), isoprene rubbers; acrylic rubbers such as polybutyl acrylic acid; and ethylene-propylene rubbers and terpolymers of ethylene-propylene-diene (EPDM). Other copolymers that may be employed in the process include acrylonitrile, methacrylonitrile, acrylic acid, methacrylic acid, maleic anhydride and N-substituted maleimide. Other vinyl monomers may be used include α-methylstyrene and p-methylstyrene. Additional modified rubber resins may include acrylonitrile-butadiene-styrene (ABS), copolymer resins of acrylonitrile-acrylic rubber-styrene (AAS) and copolymer resins of acrylonitrile-ethylenepropylene rubber-styrene (AES).

In one example, 90 g of butadiene rubber latex powder, 5 g of a selected functionalized IL, 10 g of acrylonitrile and 150 g of deionized water are mixed together. To the mixture is added 0.4 g cumen hydroperoxide and 0.01 ferrous sulfate hydrate. The mixture is heated at about 75 to 85 °C for about 5 hours. The mixture is coagulated to obtain a modified rubber polystyrene resin in a powder form.

In one aspect, the flame retarding resins may also contain a filler for improving the physical and mechanical properties of the resins. In one aspect, the filler may include glass fibers, glass flakes, glass beads, glass powders, carbon fibers, carbon flakes, talc, mica, kaolin, montmorillonite, bentonite, sepiolite, xonotlite, clay, silica, titanium oxide, carbon black, organic fillers and combinations thereof.

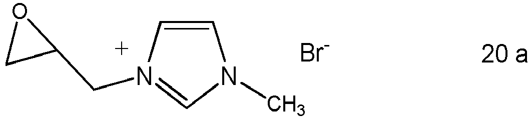

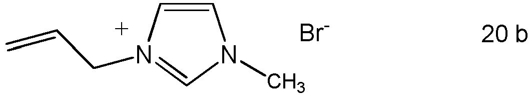

A resin containing ILs of the present application may be prepared as follows. A mixture of the epoxide **20a** in about 5% to 10% by weight, and an elastomer, about 80 to 95% by weight, containing at least acrylonitrile butadiene rubber containing a carboxyl group, and a hardening accelerator such as an organic phosphine or a phosphonium salt is combined and mixed. Optionally, aluminum hydroxide (0.1% to 5% by weight) and a filler (1% to about 5% by weight) containing talc may be added to the mixture. The mixture may be heated to a temperature of about 180 °C to 270 °C with agitation to form the desired resin. The resulting composition provides a resin that is shown to be flame resistant and the resin has sufficient flexibility and may be used effectively as an electrical insulator. The resin composition prepared according to the method may be used as adhesive insulation that is flame resistant or as printed circuit boards that are flame resistant.

Optionally, other resin additives, including polytrimethylene terephthalate based compounds such as polyethylene terephthalate, polybutylene terephthalate or nylon may be used as a resin additive in the above process to form various fibers and resins containg ILs that are flame resistant.

### Incorporation of ILs with Clays:

The clay nanomaterials may be assembled with ILs using macro-scale assembly processes, such as the layer-by-layer (LBL) assembly methods. The method involves the alternating deposisiton of components from dilute solutions or discpersions on a suitable substrate, including inorganic molecular clusters, nanoparticles, nanotubes and nanowires, nanoplates, dendrimers and clay nanosheets. See for example, P.T. Hammond, Adv. Mater. 16 (2004) 1271. The method allows the formation of multi-functional thin films.

In one example, a mixture of an IL may be combined with a synthetic clay, such as hectorite (Laponite RD) to grow several hundred-nanometers thick films. In certain aspect, the clay may be a montmorillonite or a saponite. Standard layered silicates may also be employed as the clays. The resulting film provides a highly uniform surface coverage of the IL on the substrate, and may form clay multilayers. The nature of the final sheets may depend on various parameters employed, including the adsorption time, the concentration of the IL in the misture, the amount of clay in the dispersion and the pH of the aqueous solution. Thin films of clays and ILs may also be prepared using the traditional dipping method (or dip coating method) or the monolayer deposition method as known in the art. According to the methods, formation of individual nanosheets may be used as flexible fabric, wherein the fabrics are incorporated with flame retardants.

A flame retardant montmorillonite clay may be prepared by modification of a sodium montmorillonite clay with the epoxide **20a** by an ion exchange reaction. Optionally, surface functionalization may be performed by grafting with an epoxide group containing a silane compound. The resulting flame retardant clay may be added to an epoxy resin and thermally cured to form various epoxy nanocomposites that are flame retardant.

Similarly, a flame retardant montmorillonite clay may be prepared by modification of a sodium montmorillonite clay with the IL **20b** by an ion exchange reaction. The resulting flame retardant clay may be added to an epoxy resin and thermally cured to form various epoxy nanocomposites that are flame retardant.

Molding composition containing ILs may also be prepared. A mixture of a hardener for an epoxy resin, such as phenolic novolak resin, the epoxide **20a** (about 5% to 10% by weight) and a quaternary organophosphonium satlt for catalyzing a reaction between the epoxy resin, the hardener and the epoxide **20a.** The resulting mixture may be heated to form the flame retardant molding composition that may be used for coating electronic devices.

Flame resistant polyurethanes may be prepared by mixing the epoxide **20a** (about 500 g) with diglyme (500 mL) and about 0.5% KOH. The resulting mixture is heated under vacuum, and propylene oxide (495 g) is added. A polyether polyol from bisphenol A, diethanolamine, formaldehyde, propylene oxide and a glycerol-based polyether polyol is added. Mixing and curing the resulting composition at elevated temperatures provide a foam polyurethane having flame resistant properties.

The preparation of nanocomposites comprising ILs may be performed using various methods, including the solvent intercalation route that employs swelling the layered silicates in ILs to promote the diffusion of the ILs in the clay interlayer spacing, or the melt intercalation process which is based on polymer processing in the molten state such as extrusion. See for example, Sinha Ray S, Maiti P, Okamoto M, Yamada K, Ueda K. New polylactide/layered silicate nanocomposites. 1. Preparation, characterization and properties. Macromolecules 2002; 35:3104-10; and Tanoue S, Hasook A, Iemoto Y, Unryu T. Preparation of poly(lactic acid)/poly(ethylene glycol)/organoclay nanocomposites by melt compounding. Polym Compos 2006; 27:256-63, which is incorporate herein in their entirety.

### Compounding Treatment of Polyoxymethylene with 1-Butyl-3-methylimidazolium bromide and aluminum hydroxide:

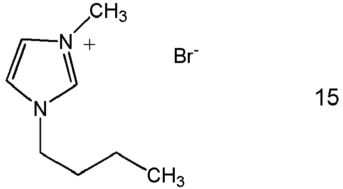

Aluminum hydroxide power (5 gm) is premixed with ionic liquid 15 (95 gm), then mixed with polyoxymethylene pellets (900 gm), and then melt-blended by a twin screw extruder at 170-185 °C with a screw rotation speed of 150-180 rpm. The extruded pellets are molded into standard bars for combustibility and mechanical performance tests through an injection-molding machine with a plasticizing temperature of 170-195 °C.

Compounding treatment of polypropylene with intumescent flame retarding system using triethylmethylphosphonium dibutyl phosphate ≥97.0% (CH).

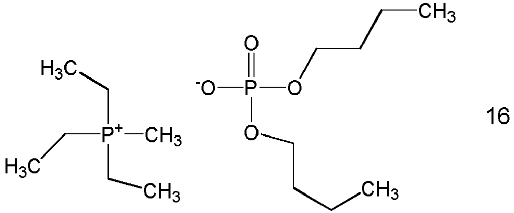

A mixture of ionic liquid **16** (2 gm), pentaerythritol (carbonization agent) (5 gm) and melamine (3 gm) are premixed and then mixed with polypropylene (90 gm). The mixture is then melt-blended by a twin screw extruder at 200 °C with a screw rotation speed of 150-180 rpm. The extruded pellets are molded into standard bars for combustibility and mechanical performance tests through an injection-molding machine with a plasticizing temperature of 230 °C.

### Treatment of PVC using IL 15 with antimony trioxide:

A mixture of IL **15** (5 gm) and antimony trioxide (2 gm) are premixed, and then mixed with polyvinyl chloride resin (93 gm). The mixture is blended and molded into required shape and dimension in a similar manner as disclosed above.

### Treatment of PVC using IL 14 and traditional brominated flame retardant tetrabromobisphenol A:

A mixture of IL **14** (3 gm), TBBPA (3 gm) are premixed, and mixed with PVC resin (94 gm). The mixture is blended and molded into required shape and dimension in a similar manner as disclosed above.

### Treatment of high density polyethylene (HDPE) with ionic tributylmethylphosphonium methyl carbonate liquid modified clay:

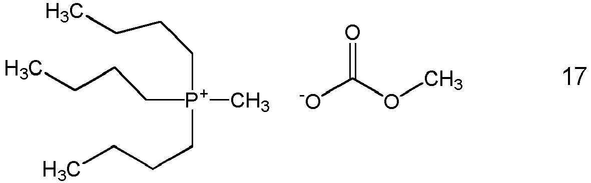

The surface of the clay is modified with ionic liquids through ion exchange reaction. HDPE (97 gm) and IL **17** modified clay (3 gm) are mixed, melt blended in ThermoHaake Rheomix with a screw speed of 60 rpm, and the mixing time for each sample is 15 min. The mixed samples are transferred to a mold and preheated at 180 °C for 5 min and then pressed at 15 MPa followed by cooling them to room temperature while maintaining the pressure for 5 min.

Treatment of polyimide 6 with ionic liquid/carbon nanotubes or ionic liquid/carbon nanofibers using 1-butylpyridinium bromide:

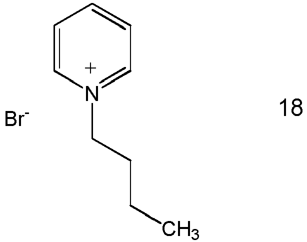

A mixture of IL **18** (3 gm) and carbon nanotubes or nanofibers (2 gm) are premixed, and then melt-blended and molded in a similar manner as disclosed above. Treatment of polystyrene via in-situ polymerization method:

A mixture of styrene (95 g), IL **15** (5 g), AIBN (0.2 g) is prepared. The mixture is stirred magnetically under nitrogen at room temperature until a homogenous mixture is formed. The mixture is heated at 90 °C for pre-polymerization until a critical viscosity of the mixture is reached. The mixture was then transferred to an oven and kept isothermally at 60 °C for 24 h and then at 80 °C for 20 h. A copolymer containing IL is obtained.

### Application IL flame retardants as a components of coating or paint layers:

Ionic liquid flame retardant **16** (5 g) is mixed with 250 ml of paint and coating materials. The resulting material is used as a heat resistant or flame resistant coating on potentially flammable surfaces. Heating of the coated materials shows that the materials are heat resistant or flame resistant to about 455 °C. The coating composition may include those formulated form modified epoxy ester resin coating, modified silicone-alkyd resin coating, specially modified silicone acrylic resin and modified silicone acrylic.

The polymers containing the ILs prepared according to the methods above, for example provides significantly improved UL94 test characteristics.

## Claims

1. Use of an ionic liquid compound as a flame retardant in a material comprising 80 to 99.9 weight percent polymer, wherein said ionic liquid compound is blended with the polymer, the ionic liquid compound having the formula: 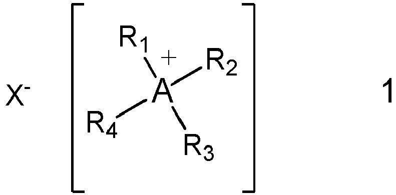 wherein A is selected from N or P; and wherein
i) when A is N, each R₁, R₂, R₃ and R₄ independently form a double bond with N and an adjacent R₁, R₂, R₃ or R₄ group or are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or wherein R₁, R₂ and R₃ together with N form a heteroaromatic or R₁ and R₂ together with N form a heterocyclic ring each unsubstituted or substituted by a group selected from halo, nitro, trifluoromethyl, trifluoromethoxy, methoxy, carboxy, -NH₂, -OH, -SH, -NHCH₃, -N(CH₃)₂, -SMe, cyano, (C₁-C₃)alkyl, aryl, (C₃-C₆)cycloalkyl, aryl(C₁-C₃)alkyl and heteroaryl;
and
ii) when A is P, R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, - OC(O)-CH=CH, -NCO, -C(O)Cl, -C(O)Br, -C(O)-imidazolyl, -CO₂(C₁-C₃)alkyl,-OC(O)CH₂C(O)CH₃ and -CH=CR₁₀CO₂(C₁-C₃)alkyl where R₁₀ is H or CH₃;
provided that:
a) when A is P, then R₁, R₂, R₃ and R₄ are not all hydroxymethyl;
b) when A is N, X⁻ is selected from the group consisting of [PF₆]⁻, [NTf₂]⁻, [BR₅R₆R₇R₈]⁻ [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻ , [CF₃CO₂]⁻ , [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, - OH, -SH, -NHCH₃, -N(CH₃)₂, -SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two nitro, methoxy, carboxy, -NH₂, -OH, -SH, -NHCH₃, -N(CH₃)₂, -SMe and cyano;
c) when A is N and at least one of R₁, R₂, R₃ and R₄ is -CH₃, then the material does not comprise of a clay; and
d) when A is P, then X⁻ is selected from the group consisting of [PF₆]⁻, [NTf₂]⁻, [BR₅R₆R₇R₈]⁻, [BF₄]⁻, OH⁻, SCN⁻, SBF₆⁻, R₉PO₄⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, [Al₂Cl₇]⁻, [AlCl₄]⁻, oxalate, dicarboxylate and tricarboxylate, formate, phosphate, I⁻ and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, - OH, -SH, -NHCH₃, -N(CH₃)₂, -SMe and cyano, and R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano; and provided that when the material is a resin and at least one of R₁, R₂, R₃ and R₄ is a -(CH₂)ₙ-H group where n is 1 to 18, then X⁻ is not SBF₆⁻, PF₆⁻, BF₄⁻, AlF₆³⁻, CF₃SO₃⁻, AsF₆⁻, B[C₆F₅]₄⁻, B[C₆H₃(C₆H₃(CF₃)₂]₄⁻, B[C₆H₅]₄⁻, TiF₆²⁻, TiCl₅⁻, SnCl₅⁻, GeF₆²⁻, SiF₆²⁻, NiF₆²⁻ or ZrF₆²⁻;
and optionally, wherein X⁻ is a group as defined above that is bonded to the polymer.

2. The use according to Claim 1, wherein A⁺ together with R₁, R₂, R₃ and R₄ form a compound selected from the group consisting of an ammonium, imidazolium, guanidinium, pyridinium, pyridazinium, 1,2,4-triazolium, triazine, phosphazenium and phosphonium.

3. The use according to Claim 1 wherein the compound is of the formula 2 or 9: 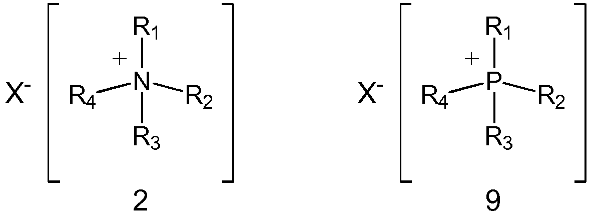
wherein for formula 2, each R₁, R₂, R₃ and R₄ independently form a double bond with N and an adjacent R₁, R₂, R₃ or R₄ group;
wherein for formula 2 and 9, R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂;
or for formula 2, wherein R₁, R₂ and R₃ together with N form a heteroaromatic or R₁ and R₂ together with N form a heterocyclic ring each unsubstituted or substituted by a group selected from halo, nitro, trifluoromethyl, trifluoromethoxy, methoxy, carboxy, -NH₂, -OH, -SH, - NHCH₃, -N(CH₃)₂, -SMe, cyano, (C₁-C₃)alkyl, aryl, (C₃-C₆)cycloalkyl, aryl(C₁-C₃)alkyl and heteroaryl; or
wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two nitro, methoxy, carboxy, -NH₂, -OH, -SH, -NHCH₃, -N(CH₃)₂, -SMe and cyano.

4. The use according to Claim 1 wherein the compound is of the formula: 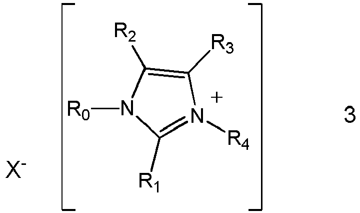 wherein:
Rₒ is selected from the group consisting of (C₁-C₅)alkyl and aryl that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; and
R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R₀, R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two nitro,, methoxy, carboxy, -NH₂, -OH, -SH, -NHCH₃, -N(CH₃)₂, -SMe and cyano.

5. The use according to Claim 1 wherein the compound is of the formula: 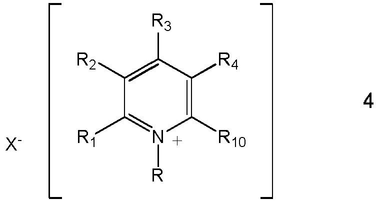
wherein R₁, R₂, R₃, R₄ and R₁₀ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂;
R is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R, R₁, R₂, R₃, R₄ and R₁₀ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two nitro, methoxy, carboxy, -NH₂, -OH, -SH, -NHCH₃, -N(CH₃)₂, -SMe and cyano.

6. The use according to Claim 1 wherein the compound is of the formula 5 or 6: 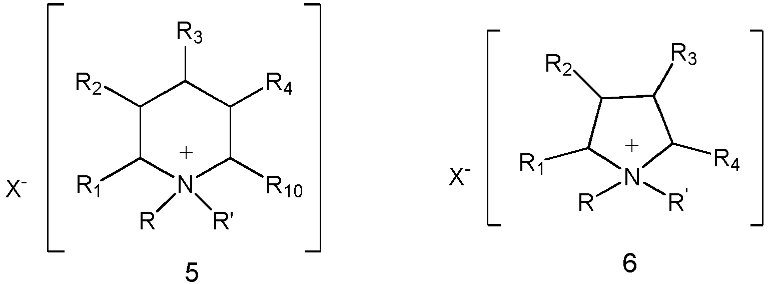
wherein R₁, R₂, R₃, R₄ and R₁₀ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R, R₁, R₂, R₃, R₄ and R₁₀ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
R and R' are independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

7. The use according to Claim 1 wherein the compound is of the formula 7 or 8: 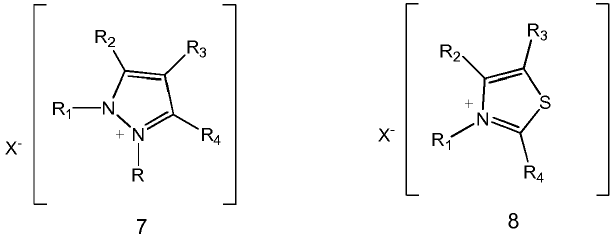 wherein:
R is selected from the group consisting of (C₁-C₅)alkyl and aryl that may be unsubstituted or substituted by one or two halo, nitro, trifluoromethyl, trifluoromethoxy, methoxy, carboxy, - NH₂, -OH, -SH, -NHCH₃, -N(CH₃)₂, cyano, -SMe, -SO₃H, -P((C₁-C₅)alkyl)₂ and -P(O)(OEt)₂; and
R₁, R₂, R₃ and R₄ are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R, R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two nitro, methoxy, carboxy, -NH₂, -OH, -SH, -NHCH₃, -N(CH₃)₂, -SMe and cyano.

8. Use of an ionic liquid compound as a flame retardant in a material comprising 80 to 99.9 weight percent polymer, wherein said ionic liquid compound is blended with the polymer, the ionic liquid compound having the formula: 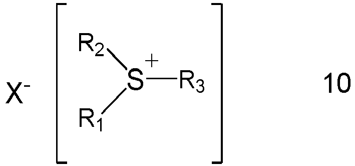
wherein R₁, R₂ and R₃ or are each independently selected from the group consisting of hydrogen, (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, cyano, -SMe, -SO₃H, -P((C₁-C₅)alkyl)₂ and -P(O)(OEt)₂; or
wherein at least one of R₁, R₂, R₃ and R₄ is selected from the group consisting of (C₁-C₁₀)alkyl substituted with one -Cl, -Br, -I, -CH=CH, -CH₂CH=CH, -epoxide, -OC(O)-CH=CH, -NCO, -C(O)Cl, -C(O)Br, -C(O)-imidazolyl, -CO₂(C₁-C₃)alkyl , -OC(O)CH₂C(O)CH₃ and -CH=CR₁₀CO₂(C₁-C₃)alkyl where R₁₀ is H or CH₃; and
X⁻ is selected from the group consisting of [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, tris(trifluoromethylsulfonyl)methide, [N(CN)₂]⁻, [CH₃CO₂]⁻, [CF₃CO₂]⁻, [NO₃]⁻, Br⁻, Cl⁻, I⁻, [Al₂Cl_{7]}⁻, [AlCl₄]⁻, oxalate, dicarboxylates and tricarboxylate, formate, phosphate and aluminate, wherein R₅, R₆, R₇ and R₈ are each independently selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, - SMe and cyano, and wherein R₉ is selected from the group consisting of (C₁-C₂₀)alkyl, aryl, (C₃-C₁₀)heterocyclyl, (C₃-C₁₀)cycloalkyl, (C₃-C₁₀)heterocyclyl(C₁-C₈)alkyl, aryl(C₁-C₈)alkyl, heteroaryl and heteroaryl(C₁-C₈)alkyl group that may be unsubstituted or substituted by one or two halo, nitro, trifluoromethyl, trifluoromethoxy, methoxy, carboxy, -NH₂, -OH, -SH, -NHCH₃, -N(CH₃)₂, -SMe and cyano.

9. The use of any one of Claims 1 to 8, further comprising contacting or formulating the ionic liquid with a second different ionic liquid.

10. The use of any one of Claims 1 to 9, further comprising contacting or formulating the material with an ionic liquid in combination with an agent selected from the group consisting of a mineral flame retardant, a halogenated flame retardant, a phosphorus based flame retardant, a nitrogen based flame retardant, a silicon based flame retardants and nanometric particles, and combinations thereof.

## Patentansprüche

1. Verwendung einer ionischen flüssigen Verbindung als Flammschutzmittel in einem Material, das 80 bis 99,9 Gewichtsprozent Polymer umfasst, wobei die ionische flüssige Verbindung mit dem Polymer gemischt wird, wobei die ionische flüssige Verbindung die folgende Formel hat: 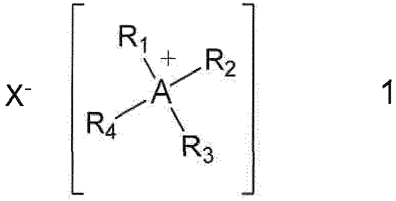 wobei A aus N oder P ausgewählt ist; und wobei
i) wenn A N ist, R₁, R₂, R₃ und R₄ jeweils unabhängig eine Doppelbindung mit N und einer benachbarten R₁-, R₂-, R₃- oder R₄-Gruppe bilden oder jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Heteroaryl und Heteroary)(C₁-C₈)alkyl-Gruppe, die unsubstituiert sein kann, oder substituiert mit einem oder zwei Halogen, Nitro, Trifluormethyl, Trifluormethoxy, Methoxy, Carboxy, -NH₂, - OH, -SH, -NHCH₃, -N(CH₃)₂, Cyano, -SMe, -SO₃H, -P((C₁-C₅)-Alkyl)₂ und -P(O)(OEt)₂; oder wobei R₁, R₂ und R₃ zusammen mit N einen heteroaromatischen bilden oder R₁ und R₂ zusammen mit N einen heterocyclischen Ring bilden, jeweils unsubstituiert oder substituiert mit einer Gruppe ausgewählt aus Halogen, Nitro, Trifluormethyl, Trifluormethoxy, Methoxy, Carboxy, -NH₂, -OH, -SH, -NHCH₃, -N(CH₃)₂, -SMe, Cyano, (C₁-C₃)-Alkyl, Aryl, (C₃-C₆)-Cycloalkyl, Aryl(C₁-C₃)-Alkyl und Heteroaryl;
und
ii) wenn A P ist, R₁, R₂, R₃ und R₄ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)alkyl, Aryl(C₁-C₈)alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, - OH, -SH, -NHCH₃, -N(CH₃)₂, Cyano, -SMe, -SO₃H, -P((C₁-C₅)-Alkyl)₂ und -P(O)(OEt)₂; oder
wobei mindestens eins von R₁, R₂, R₃ und R₄ ausgewählt ist aus der Gruppe bestehend aus (C₁-C₁₀)-Alkyl, substituiert mit einem -Cl, -Br, -I, -CH=CH, -CH₂CH=CH, - Epoxid, -OC(O)-CH=CH, -NCO, -C(O)Cl, -C(O)Br, -C(O)-Imidazolyl, -CO₂(C₁-C₃)-Alkyl, - OC(O)CH₂C(O)CH₃ und -CH=CR₁₀CO₂(C₁-C₃)-Alkyl, wobei R₁₀ H oder CH₃ ist;
unter der Voraussetzung, dass:
a) wenn A P ist, R₁, R₂, R₃ und R₄ dann nicht alle Hydroxymethyl sind;
b) wenn A N ist, X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Nitro, Methoxy, Carboxy, -NH₂, -OH, -SH, -NHCH₃, -N(CH₃)₂, -SMe und Cyano;
c) wenn A N ist und mindestens eins von R₁, R₂, R₃ und R₄ -CH₃ ist, das Material dann keinen Ton umfasst; und
d) wenn A P ist, X⁻ dann ausgewählt ist aus der Gruppe bestehend aus [PF₆]⁻, [NTf₂]⁻, [BR₅R₆R₇R₈]⁻, [BF₄]⁻, OH⁻, SCN⁻, SBF₆⁻, R₉PO₄⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, [Al₂Cl₇]⁻, [AlCl₄]⁻, Oxalat, Dicarboxylat und Tricarboxylat, Formiat, Phosphat, I⁻ und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, -SMe und Cyano, und R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, -SMe und Cyano; und unter der Voraussetzung, dass, wenn das Material ein Harz ist und mindestens eins von R₁, R₂, R₃ und R₄ eine -(CH₂)ₙ-H-Gruppe ist, bei der n 1 bis 18 ist, X⁻ dann nicht SBF₆⁻, PF₆⁻, BF₄⁻, AlF₆³⁻, CF₃SO₃⁻, AsF₆⁻, B[C₆F₅]₄⁻, B[C₆H₃(C₆H₃(CF₃)₂]₄⁻, B[C₆H₅]₄⁻, TiF₆²⁻, TiCl₅⁻, SnCl₅⁻, GeF₆²⁻, SiF₆²⁻, NiF₆²⁻ oder ZrF₆²⁻ ist;
und wobei X⁻ optional eine Gruppe wie oben definiert ist, die an das Polymer gebunden ist.

2. Die Verwendung gemäß Anspruch 1, wobei A⁺ zusammen mit R₁, R₂, R₃ und R₄ eine Verbindung bildet, die ausgewählt ist aus der Gruppe bestehend aus einem Ammonium, Imidazolium, Guanidinium, Pyridinium, Pyridazinium, 1,2,4-Triazolium, Triazin, Phosphazenium und Phosphonium.

3. Die Verwendung gemäß Anspruch 1, wobei die Verbindung Formel 2 oder 9 entspricht: 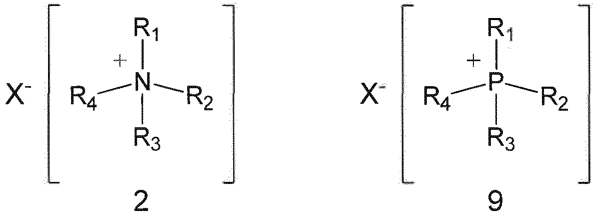
wobei für Formel 2, R₁, R₂, R₃ und R₄ jeweils unabhängig eine Doppelbindung mit N und einer benachbarten R₁, R₂, R₃ oder R₄-Gruppe bilden;
wobei für Formel 2 und 9 R₁, R₂, R₃ und R₄ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, Cyano, -SMe, -SO₃H, -P((C₁-C₅)-Alkyl)₂ und -P(O)(OEt)₂;
oder, für Formel 2, wobei R₁, R₂ und R₃ zusammen mit N heteroaromatischen bilden oder R₁ und R₂ zusammen mit N einen heterocyclischen Ring bilden, jeweils unsubstituiert oder substituiert mit einer Gruppe ausgewählt aus Halogen, Nitro, Trifluormethyl, Trifluormethoxy, Methoxy, Carboxy, -NH₂, - OH, -SH, -NHCH₃, -N(CH₃)₂, -SMe, Cyano, (C₁-C₃)-Alkyl, Aryl, (C₃-C₆)-Cycloalkyl, Aryl(C₁-C₃)-Alkyl und Heteroaryl; oder
wobei mindestens ein von R₁, R₂, R₃ und R₄ ausgewählt ist aus der Gruppe bestehend aus (C₁-C₁₀)-Alkyl, substituiert mit einem -Cl, -Br, -I, -CH=CH, -CH₂CH=CH, -Epoxid, -OC(O)-CH=CH, -NCO, - C(O)Cl, -C(O)Br, -C(O)-Imidazolyl, -CO₂(C₁-C₃)-Alkyl , -OC(O)CH₂C(O)CH₃ und -CH=CR₁₀)CO₂(C₁-C₃)-Alkyl wobei R₁₀ H oder CH₃ ist; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalate, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Nitro, Methoxy, Carboxy, -NH₂, -OH, -SH, -NHCH₃, -N(CH₃)₂, -SMe und Cyano.

4. Die Verwendung gemäß Anspruch 1, wobei die Verbindung der folgenden Formel entspricht: 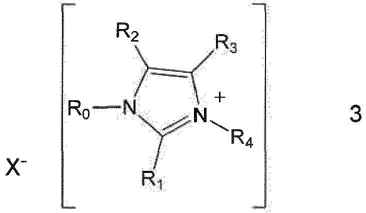 wobei:
Rₒ ausgewählt ist aus der Gruppe bestehend aus (C₁-C₅)-Alkyl und Aryl, das unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, Cyano, -SMe, -SO₃H, -P((C₁-C₅)-Alkyl)₂ und -P(O)(OEt)₂; und
R₁, R₂, R₃ und R₄ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, - NHCH₃, -N(CH₃)₂, Cyano, -SMe, -SO₃H, -P((C₁-C₅)-Alkyl)₂ und -P(O)(OEt)₂; oder
wobei mindestens ein von R₀, R₁, R₂, R₃ und R₄ ausgewählt ist aus der Gruppe bestehend aus (C₁-C₁₀)-Alkyl, substituiert mit einem -Cl, -Br, -I, -CH=CH, -CH₂CH=CH, -epoxide, -OC(O)-CH=CH, -NCO, - C(O)Cl, -C(O)Br, -C(O)-Imidazolyl, -CO₂(C₁-C₃)-Alkyl , -OC(O)CH₂C(O)CH₃ und -CH=CR₁₀CO₂(C₁-C₃)-Alkyl, wobei R₁₀ H oder CH₃ ist; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Nitro, Methoxy, Carboxy, -NH₂, -OH, -SH, -NHCH₃, -N(CH₃)₂, -SMe und Cyano.

5. Die Verwendung gemäß Anspruch 1, wobei die Verbindung der folgenden Formel entspricht: 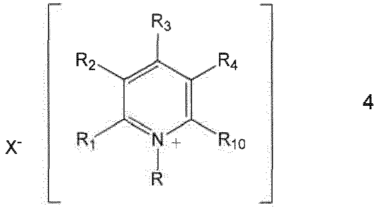
wobei R₁, R₂, R₃, R₄ und R₁₀ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, Cyano, -SMe, -SO₃H, -P((C₁-C₅)-Alyl)₂ und -P(O)(OEt)₂;
R ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-AlkylGruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, Cyano, -SMe, - SO₃H, -P((C₁-C₅)-Alkyl)₂ und -P(O)(OEt)₂; oder
wobei mindestens ein von R, R₁, R₂, R₃, R₄ und R₁₀ ausgewählt ist aus der Gruppe bestehend aus (C₁-C₁₀)-Alkyl, substituiert mit einem -Cl, -Br, -I, -CH=CH, -CH₂CH=CH, -Epoxid, -OC(O)-CH=CH, -NCO, - C(O)Cl, -C(O)Br, -C(O)-Imidazolyl, -CO₂(C₁-C₃)-Alkyl, -OC(O)CH₂C(O)CH₃ und -CH=CR₁₀CO₂(C₁-C₃)-Alkyl, wobei R₁₀ H oder CH₃ ist; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Nitro, Methoxy, Carboxy, -NH₂, -OH, -SH, -NHCH₃, -N(CH₃)₂, -SMe und Cyano.

6. Die Verwendung gemäß Anspruch 1, wobei die Verbindung der Formel 5 oder 6 entspricht: 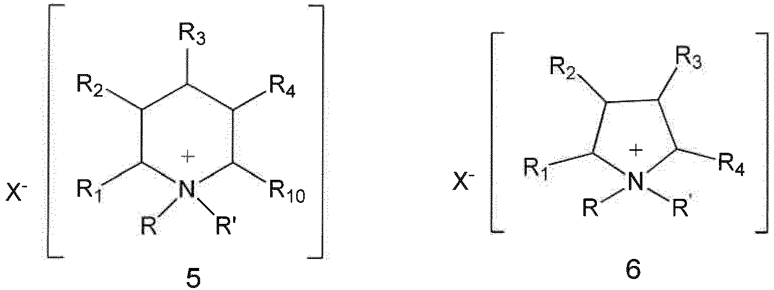
wobei R₁, R₂, R₃, R₄ und R₁₀ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, Cyano, -SMe, -SO₃H, -P((C₁-C₅)-Alkyl)₂ und -P(O)(OEt)₂; oder
wobei mindestens ein von R, R₁, R₂, R₃, R₄ und R₁₀ ausgewählt ist aus der Gruppe bestehend aus (C₁-C₁₀)-Alkyl, substituiert mit einem -Cl, -Br, -I, -CH=CH, -CH₂CH=CH, -Epoxid, -OC(O)-CH=CH, -NCO, - C(O)Cl, -C(O)Br, -C(O)-Imidazolyl, -CO₂(C₁-C₃)-Alkyl , -OC(O)CH₂C(O)CH₃ und -CH=CR₁₀CO₂(C₁-C₃)-Alkyl, wobei R₁₀ H oder CH₃ ist; und
R und R' unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, Cyano, -SMe, -SO₃H, -P((C₁-C₅)-Alkyl)₂ und -P(O)(OEt)₂; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano.

7. Die Verwendung gemäß Anspruch 1 wobei die Verbindung der Formel 7 oder 8 entspricht: 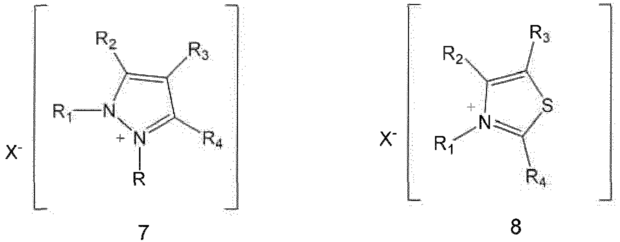 wobei:
R ausgewählt ist aus der Gruppe bestehend aus (C₁-C₅)-Alkyl und Aryl, das unsubstituiert sein kann, oder substituiert mit einem oder zwei Halogen, Nitro, Trifluormethyl, Trifluormethoxy, Methoxy, Carboxy, -NH₂, -OH, -SH, -NHCH₃, -N(CH₃)₂, Cyano, -SMe, -SO₃H, -P((C₁-C₅)-Alkyl)₂ und -P(O)(OEt)₂; und
R₁, R₂, R₃ und R₄ jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-A)kyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, -NH₂, -OH, -SH, - NHCH₃, -N(CH₃)₂, Cyano, -SMe, -SO₃H, -P((C₁-C₅)-Alkyl)₂ und -P(O)(OEt)₂; oder
wobei mindestens ein von R, R₁, R₂, R₃ und R₄ ausgewählt ist aus der Gruppe bestehend aus (C₁-C₁₀)-Alkyl substituted with one -Cl, -Br, -I, -CH=CH, -CH₂CH=CH, -epoxide, -OC(O)-CH=CH, -NCO, - C(O)Cl, -C(O)Br, -C(O)-Imidazolyl, -CO₂(C₁-C₃)-Alkyl , -OC(O)CH₂C(O)CH₃ und -CH=CR₁₀CO₂(C₁-C₃)-Alkyl, wobei R₁₀ H oder CH₃ ist; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf⁻, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Nitro, Methoxy, Carboxy, -NH₂, -OH, -SH, -NHCH₃, -N(CH₃)₂, -SMe und Cyano.

8. Verwendung einer ionischen flüssigen Verbindung als Flammschutzmittel in einem Material, das 80 bis 99,9 Gewichtsprozent Polymer umfasst, wobei die ionische flüssige Verbindung mit dem Polymer gemischt wird, wobei die ionische flüssige Verbindung die folgende Formel hat: 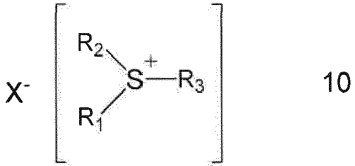
wobei R₁, R₂ und R₃ oder jeweils unabhängig ausgewählt sind aus der Gruppe bestehend aus Wasserstoff, (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, Cyano, -SMe, -SO₃H, -P((C₁-C₅)-Alkyl)₂ und -P(O)(OEt)₂; oder
wobei mindestens ein von R₁, R₂, R₃ und R₄ ausgewählt ist aus der Gruppe bestehend aus (C₁₋C₁₀)-Alkyl, substituiert mit einem -Cl, -Br, -I, -CH=CH, -CH₂CH=CH, -Epoxid, -OC(O)-CH=CH, -NCO, - C(O)Cl, -C(O)Br, -C(O)-lmidazolyl, -CO₂(C₁-C₃)-Alkyl, -OC(O)CH₂C(O)CH₃ und -CH=CR₁₀CO₂(C₁-C₃)-Alkyl, wobei R₁₀ H oder CH₃ ist; und
X⁻ ausgewählt ist aus der Gruppe bestehend aus [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf⁻, Tris(trifluormethylsulfonyl)methid, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, Oxalat, Dicarboxylaten und Tricarboxylat, Formiat, Phosphat und Aluminat, wobei R₅, R₆, R₇ und R₈ jeweils unabhängig ausgewählt sind aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano, und wobei R₉ ausgewählt ist aus der Gruppe bestehend aus (C₁-C₂₀)-Alkyl, Aryl, (C₃-C₁₀)-Heterocyclyl, (C₃-C₁₀)-Cycloalkyl, (C₃-C₁₀)-Heterocyclyl(C₁-C₈)-Alkyl, Aryl(C₁-C₈)-Alkyl, Heteroaryl und Heteroaryl(C₁-C₈)-Alkyl-Gruppe, die unsubstituiert sein kann, oder substituiert mit einem oder zwei Halogen, Nitro, Trifluormethyl, Trifluormethoxy, Methoxy, Carboxy, - NH₂, -OH, -SH, -NHCH₃, -N(CH₃)₂, -SMe und Cyano.

9. Die Verwendung von einem der Ansprüche 1 bis 8, außerdem umfassend das in-Kontakt-Bringen oder Formulieren der ionischen Flüssigkeit mit einer zweiten unterschiedlichen ionischen Flüssigkeit.

10. Die Verwendung von einem der Ansprüche 1 bis 9, außerdem umfassend das in-Kontakt-Bringen oder Formulieren des Materials mit einer ionischen Flüssigkeit in Kombination mit einem Agens, ausgewählt aus der Gruppe bestehend aus einem mineralischen Flammschutzmittel, einem halogenierten Flammschutzmittel, einem Phosphor-basierten Flammschutzmittel, einem Stickstoffbasierten Flammschutzmittel, einem Silicium-baiserten Flammschutzmittel und nanometrischen Partikeln, und Kombinationen davon.

## Revendications

1. Utilisation d'un composé liquide ionique en tant que retardateur de flamme dans un matériau comprenant 80 à 99,9 pour cent en poids de polymère, dans lequel ledit composé liquide ionique est mélangé au polymère, le composé liquide ionique ayant la formule : 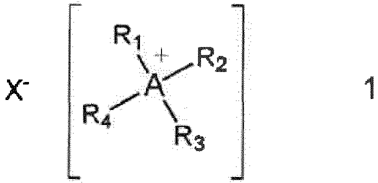 dans laquelle A est choisi parmi N ou P ; et dans laquelle
i) lorsque A est N, chacun de R₁, R₂, R₃ et R₄ forme indépendamment une double liaison avec N et un groupe R₁, R₂, R₃ ou R₄ adjacent ou sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, cyano, -SMe, -SO₃H, -P (alkyle (en C₁ à C₅))₂ et -P(O)(OEt)₂ ; ou dans laquelle R₁, R₂ et R₃ conjointement avec N forment un hétéroaromatique ou R₁ et R₂ conjointement avec N forment un cycle hétérocyclique chacun non substitué ou substitué par un groupe choisi parmi halogéno, nitro, trifluorométhyle, trifluorométhoxy, méthoxy, carboxy, - NH₂, -OH, -SH, -NHCH₃, -N(CH₃)₂, -SMe, cyano, alkyle (en C₁ à C₃), aryle, cycloalkyle (en C₃ à C₆), arylalkyle (en C₁ à C₃) et hétéroaryle ;
et
ii) lorsque A est P, R₁, R₂, R₃ et R₄ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H,-P (alkyle (en C₁ à C₅))₂ et -P(O)(OEt)₂ ; ou
dans laquelle au moins l'un de R₁, R₂, R₃ et R₄ est choisi dans le groupe consistant en alkyle (en C₁ à C₁₀) substitué par un -Cl, -Br, -I, -CH=CH, -CH₂CH=CH, - époxyde, -OC(O)-CH=CH, -NCO, -C(O)Cl, -C(O)Br, -C(O)-imidazolyle, -CO₂alkyle (en C₁ à C₃), -OC(O)CH₂C(O)CH₃ et -CH=CR₁₀CO₂alkyle (en C₁ à C₃) où R₁₀ est H ou CH₃ ;
à condition que :
a) lorsque A est P, alors R₁, R₂, R₃ et R₄ ne sont pas tous hydroxyméthyle ;
b) lorsque A est N, X⁻ est choisi dans le groupe consistant en [PF₆]⁻, [NTf₂]⁻, [BR₅R₆R₇R8]⁻, [BF₄]⁻, OH⁻, SCN⁻, SbF6⁻, R₉PO₄⁻, R₉SO₂⁻, R₉SO₃⁻, R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, -SMe et cyano, et dans laquelle R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux nitro, méthoxy, carboxy, -NH₂, -OH, -SH, -NHCH₃, -N(CH₃)₂, -SMe et cyano ;
c) lorsque A est N et au moins l'un de R₁, R₂, R₃ et R₄ est -CH₃, alors le matériau ne comprenne pas d'argile ; et
d) lorsque A est P, alors X⁻ soit choisi dans le groupe consistant en [PF₆]⁻, [NTf₂]⁻, [BR₅R₆R₇R₈]⁻, [BF₄]⁻, OH⁻, SCN⁻, SBF₆⁻, R₉PO₄⁻, R₉SO₃⁻, R₉SO₄⁻ , OTf⁻, tris(trifluorométhylsulfonyl)méthylure, [N(CN)₂]⁻, [CH₃CO₂]⁻, [CF₃CO₂]⁻, [NO₃]⁻, [Al₂Cl₇]⁻, [AlCl₄]⁻, oxalate, dicarboxylate et tricarboxylate, formiate, phosphate, I⁻ et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, -SMe et cyano, et R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, -SMe et cyano ; et à condition que lorsque le matériau est une résine et au moins l'un de R₁, R₂, R₃ et R₄ est un groupe -(CH₂)ₙ-H où n vaut 1 à 18, alors X⁻ ne soit pas SBF₆⁻, PF₆⁻, BF₄⁻, AlF₆³⁻, CF₃SO₃⁻, AsF₆⁻, B[C₆F₅]₄⁻, B[C₆H₃(C₆H₃(CF₃)₂]₄⁻, B[C₆H₅]₄⁻, TiF₆²⁻, TiCl₅⁻, SnCl₅⁻, GeF₆²⁻, SiF₆²⁻, NiF₆²⁻ ou ZrF₆²⁻ ; et facultativement, dans laquelle X⁻ est un groupe tel que défini ci-dessus qui est lié au polymère.

2. Utilisation selon la revendication 1, dans laquelle A⁺ ensemble avec R₁, R₂, R₃ et R₄ forment un composé choisi dans le groupe consistant en un ammonium, imidazolium, guanidinium, pyridinium, pyridazinium, 1,2,4-triazolium, triazine, phosphazénium et phosphonium.

3. Utilisation selon la revendication 1, dans laquelle le composé est de formule 2 ou 9 : 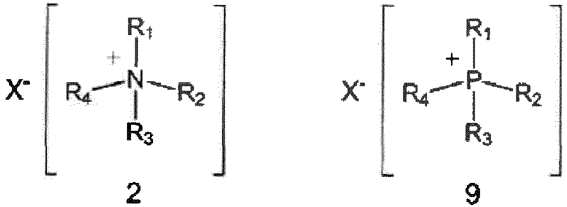
dans laquelle pour la formule 2, chaque R₁, R₂, R₃ et R₄ forme indépendamment une double liaison avec N et un groupe R₁, R₂, R₃ ou R₄ adjacent ;
dans laquelle pour la formule 2 et la formule 9, R₁, R₂, R₃ et R₄ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, - NHCH₃, -N(CH₃)₂, cyano, -SMe, -SO₃H, -P(alkyle (en C₁ à C₅))₂ et -P(O)(OEt)₂ ;
ou pour la formule 2, dans laquelle R₁, R₂ et R₃ conjointement avec N forment un hétéroaromatique ou R₁ et R₂ conjointement avec N forment un cycle hétérocyclique chacun non substitué ou substitué par un groupe choisi parmi halogéno, nitro, trifluorométhyle, trifluorométhoxy, méthoxy, carboxy, -NH₂, -OH, -SH, - NHCH₃, -N(CH₃)₂, -SMe, cyano, alkyle (en C₁ à C₃), aryle, cycloalkyle (en C₃ à C₆), arylalkyle (en C₁ à C₃) et hétéroaryle ; ou
dans laquelle au moins l'un de R₁, R₂, R₃ et R₄ est choisi dans le groupe consistant en alkyle (en C₁ à C₁₀) substitué par un -Cl, -Br, -I, -CH=CH, -CH₂CH=CH, - époxyde, -OC(O)-CH=CH, -NCO, -C(O)Cl, -C(O)Br, -C(O)-imidazolyle, -CO₂alkyle (en C₁ à C₃), -OC(O)CH₂C(O)CH₃ et -CH=CR₁₀CO₂alkyle (en C₁ à C₃) où R₁₀ est H ou CH₃ ; et
X⁻ est choisi dans le groupe consistant en [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻ , R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈) arylalkyle (en C₁ à C₈), hétéroaryle et hétéroarylalkyle (en C₁ à C₈) qui peut être non substitué ou substitué par un ou deux nitro, méthoxy, carboxy, -NH₂, -OH, -SH, -NHCH₃, -N(CH₃)₂, -SMe et cyano.

4. Utilisation selon la revendication 1, dans laquelle le composé est de formule : 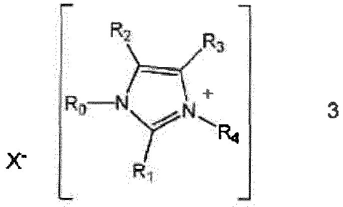 dans laquelle :
R₀ est choisi dans le groupe consistant en alkyle (en C₁ à C₅) et aryle qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H, - P (alkyle (en C₁ à C₅))₂ et -P(O)(OEt)₂ ; et
R₁, R₂, R₃ et R₄ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, cyano, -SMe, -SO₃H, -P(alkyle (en C₁ à C₅))₂ et -P(O)(OEt)₂ ; ou
dans laquelle au moins l'un de R₀, R₁, R₂, R₃ et R₄ est choisi dans le groupe consistant en alkyle (en C₁ à C₁₀) substitué par un -Cl, -Br, -I, -CH=CH, -CH₂CH=CH, - époxyde, -OC(O)-CH=CH, -NCO, -C(O)Cl, -C(O)Br, -C(O)-imidazolyle, -CO₂alkyle (en C₁ à C₃), -OC(O)CH₂C(O)CH₃ et -CH=CR₁₀CO₂alkyle (en C₁ à C₃) où R₁₀ est H ou CH₃ ; et
X⁻ est choisi dans le groupe consistant en [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R9SO₃⁻, R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₃), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux nitro, méthoxy, carboxy, -NH₂, -OH, -SH, -NHCH₃, -N(CH₃)₂, -SMe et cyano.

5. Utilisation selon la revendication 1, dans laquelle le composé est de formule : 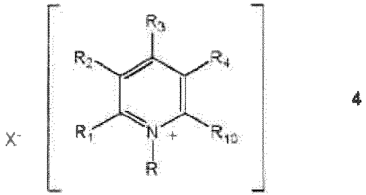
dans laquelle R₁, R₂, R₃, R₄ et R₁₀ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H, - P (alkyle (en C₁ à C₅))₂ et -P(O)(OEt)₂ ;
R est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, cyano, -SMe, -SO₃H, -P(alkyle (en C₁ à C₅))₂ et -P(O)(OEt)₂ ; ou
dans laquelle au moins l'un de R, R₁, R₂, R₃, R₄ et R₁₀ est choisi dans le groupe consistant en alkyle (en C₁ à C₁₀) substitué par un -Cl, -Br, -I, -CH=CH, - CH₂CH=CH, -époxyde, -OC(O)-CH=CH, -NCO, -C(O)Cl, - C(O)Br, -C(O)-imidazolyle, -CO₂alkyle (en C₁ à C₃), - OC(O)CH₂C(O)CH₃ et -CH=CR₁₀CO₂alkyle (en C₁ à C₃) où R₁₀ est H ou CH₃ ; et
X⁻ est choisi dans le groupe consistant en [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle Rg est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux nitro, méthoxy, carboxy, -NH₂, -OH, -SH, -NHCH₃, -N(CH₃)₂, -SMe et cyano.

6. Utilisation selon la revendication 1, dans laquelle le composé est de formule 5 ou 6 : 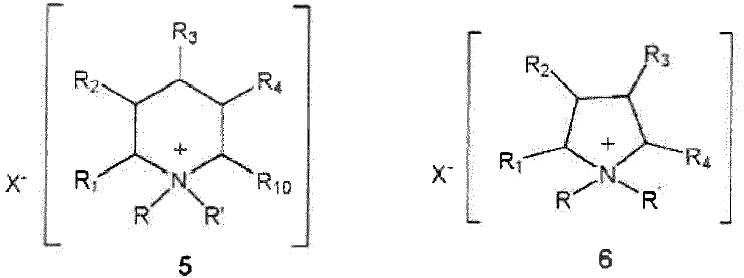
dans laquelle R₁, R₂, R₃, R₄ et R₁₀ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H, - P (alkyle (en C₁ à C₅))₂ et -P(O)(OEt)₂ ; ou
dans laquelle au moins l'un de R, R₁, R₂, R₃, R₄ et R₁₀ est choisi dans le groupe consistant en alkyle (en C₁ à C₁₀) substitué par un -Cl, -Br, -I, -CH=CH, - CH₂CH=CH, -époxyde, -OC(O)-CH=CH, -NCO, -C(O)Cl, - C(O)Br, -C(O)-imidazolyle, -CO₂alkyle (en C₁ à C₃), - OC(O)CH₂C(O)CH₃ et -CH=CR₁₀CO₂alkyle (en C₁ à C₃) où R₁₀ est H ou CH₃ ; et
R et R' sont indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H, - P (alkyle (en C₁ à C₅))₂ et -P(O)(OEt)₂ ; et
X⁻ est choisi dans le groupe consistant en [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf⁻, tris (trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, -SMe et cyano.

7. Utilisation selon la revendication 1, dans laquelle le composé est de formule 7 ou 8 : 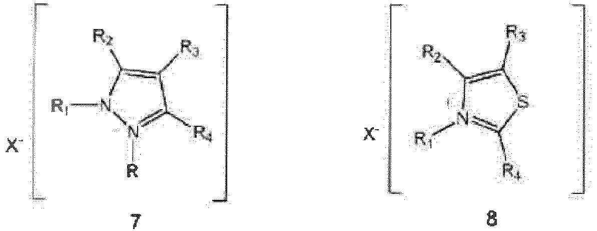 dans laquelle :
R est choisi dans le groupe consistant en alkyle (en C₁ à C₅) et aryle qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, cyano, -SMe, -SO₃H, - P(alkyle (en C₁ à C₅))₂ et -P(O)(OEt)₂ ; et
R₁, R₂, R₃ et R₄ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno nitro, trifluorométhyle, trifluorométhoxy, méthoxy, carboxy, -NH₂, -OH, -SH, -NHCH₃, -N(CH₃)₂, cyano, -SMe, -SO₃H, -P(alkyle (en C₁ à C₅))₂ et -P(O)(OEt)₂ ; ou
dans laquelle au moins l'un de R, R₁, R₂, R₃ et R₄ est choisi dans le groupe consistant en alkyle (en C₁ à C₁₀) substitué par un -Cl, -Br, -I, -CH=CH, -CH₂CH=CH, - époxyde, -OC(O)-CH=CH, -NCO, -C(O)Cl, -C(O)Br, -C(O)-imidazolyle, -CO₂alkyle (en C₁ à C₃), -OC(O)CH₂C(O)CH₃ et -CH=CR₁₀CO₂alkyle (en C₁ à C₃) où R₁₀ est H ou CH₃ ; et
X⁻ est choisi dans le groupe consistant en [PF₆]⁻, [NTf₂]⁻, [BR₅R₆R₇R₈]⁻, [BF₄]⁻, OH⁻, SCN⁻, SbF₆⁻, R₉PO₄⁻, R₉SO₂⁻, R₉SO₃⁻, R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₃, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle R₉ est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀)alkyle (en C₁ à C₈), arylalkyle (en C₁ à C₈), hétéroaryle et hétéroarylalkyle (en C₁ à C₈) qui peut être non substitué ou substitué par un ou deux nitro, méthoxy, carboxy, -NH₂, -OH, -SH, -NHCH₃, -N(CH₃)₂, -SMe et cyano.

8. Utilisation d'un composé liquide ionique comme retardateur de flamme dans un matériau comprenant 80 à 99,9 pour cent en poids de polymère, dans laquelle ledit composé liquide ionique est mélangé avec le polymère, le composé liquide ionique ayant la formule : 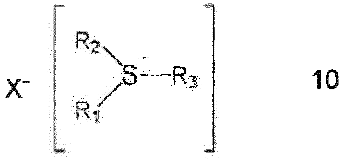
dans laquelle R₁, R₂ et R₃ sont chacun indépendamment choisis dans le groupe consistant en hydrogène, un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno nitro, trifluorométhyle, trifluorométhoxy, méthoxy, carboxy, - NH₂, -OH, -SH, -NHCH₃, -N(CH₃)₂, cyano, -SMe, -SO₃H, - P (alkyle (en C₁ à C₅))₂ et -P(O)(OEt)₂ ; ou
dans laquelle au moins l'un de R₁, R₂, R₃ et R₄ est choisi dans le groupe consistant en alkyle (en C₁ à C₁₀) substitué par un -Cl, -Br, -I, -CH=CH, -CH₂CH=CH, - époxyde, -OC(O)-CH=CH, -NCO, -C(O)Cl, -C(O)Br, -C(O)-imidazolyle, -CO₂alkyle (en C₁ à C₃), -OC(O)CH₂C(O)CH₃ et -CH=CR₁₀CO₂alkyle (en C₁ à C₃) où R₁₀ est H ou CH₃ ; et
X⁻ est choisi dans le groupe consistant en [PF₆]⁻, [NTf₂]⁻, [BR₅R₆R₇R₈]⁻, [BF₄]⁻, OH-, SCN⁻, SbF₆⁻, R₉PO₄⁻, R₉SO₂⁻, R₉SO₃⁻, R₉SO₄⁻, OTf⁻, tris(trifluorométhylsulfonyl)méthylure, [N(CN)₂]⁻, [CH₃CO₂]⁻, [CF₃CO₂]⁻, [NO₃]⁻, Br⁻, Cl⁻, I⁻, [Al₂Cl₇]⁻, [AlCl₄]⁻, oxalate, dicarboxylates et tricarboxylate, formiate, phosphate et aluminate, dans laquelle R₅, R₆, R₇ et R₈ sont chacun indépendamment choisis dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₃), hétéroaryle et hétéroarylalkyle (en C₁ à C₈) qui peut être non substitué ou substitué par un ou deux halogéno, nitro, trifluorométhyle, trifluorométhoxy, méthoxy, carboxy, - NH₂, -OH, -SH, -NHCH₃, -N(CH₃)₂, -SMe et cyano, et dans laquelle Rg est choisi dans le groupe consistant en un groupe alkyle (en C₁ à C₂₀), aryle, hétérocyclyle (en C₃ à C₁₀), cycloalkyle (en C₃ à C₁₀), hétérocyclyl (en C₃ à C₁₀) alkyle (en C₁ à C₈), arylalkyle (en C₁ à C₈), hétéroaryle et hétéroarylalkyle (en C₁ à C₈) qui peut être non substitué ou substitué par un ou deux halogéno, nitro, trifluorométhyle, trifluorométhoxy, méthoxy, carboxy, -NH₂, -OH, -SH, -NHCH₃, -N(CH₃)₂, -SMe et cyano.

9. Utilisation selon l'une quelconque des revendications 1 à 8, comprenant en outre la mise en contact ou la formulation du liquide ionique avec un second liquide ionique différent.

10. Utilisation selon l'une quelconque des revendications 1 à 9, comprenant en outre la mise en contact ou la formulation du matériau avec un liquide ionique en combinaison avec un agent choisi dans le groupe consistant en un retardateur de flamme minéral, un retardateur de flamme halogéné, un retardateur de flamme à base de phosphore, un retardateur de flamme à base d'azote, un retardateur de flamme à base de silicium et des particules nanométriques, et leurs combinaisons.
